# EUROPEAN PATENT SPECIFICATION

(11) **EP 2 863 889 B1**
(45) Date of publication and mention of the grant of the patent: **05.12.2018**
(21) Application number: 13810830.3
(22) Date of filing: 17.06.2013
(51) Int. Cl.: A61K 9/107, A23L 29/212, A23L 29/262

(54) **EMULSIFIER SYSTEM IN THE FORM OF A PASTE**
EMULGATORSYSTEM IN FORM EINER PASTE
SYSTÈME ÉMULSIFIANT SOUS LA FORME D'UNE PÂTE

(30) Priority: 25.06.2012 SE 1250678
(43) Date of publication of application: 29.04.2015
(73) Proprietor: Lyckeby Culinar AB, 290 34 Fjälkinge (SE)
(72) Inventor: LUNDBERG, Jan-Olof, S-212 28 Malmö (SE)
(74) Representative: AWA Sweden AB
(86) International application number: PCT/SE2013/050708
(87) International publication number: WO 2014/003634

(56) References cited:
- EP-A2- 0 714 609
- WO-A1-2007/112504
- WO-A1-2007/123466
- WO-A1-2012/082065
- WO-A1-2012/099527
- WO-A1-2012/099527
- US-A- 4 980 193
- US-A- 5 676 994
- DONG-HO WOO: "STABILIZATION OF THE EMULSION PREPARED WITH DIETARY FIBER FROM CORN HULL", FOOD SCIENCE AND BIOTECHNOLOGY, THE KOREA SOC. OF FOOD SCIENCE AND TECHNOLOGY, HEIDELBERG, vol. 10, no. 4, 1 January 2001 (2001-01-01), pages 348-353, XP009048544, ISSN: 1226-7708

## Description

### Technical Field of the Invention

The present invention relates to an emulsifier system comprising a fiber system including cellulose, hemicellulose, starch and oil, wherein the fiber part of the fiber system is insoluble in water and oil.

### Background Art

The fact that there are different types of emulsifiers has been known for many years. In the food industry many types of such emulsifiers are used. One example is margarine, which is an emulsion of water in oil. Emulsions can also be found in e.g. milk products and ice cream products. Sausage emulsions are other examples.

Furthermore, there are vegetable fibrous materials on the market today. A flavour release material is e.g. described in WO07123466 A1. Flavour release materials comprise a vegetable fibrous material with natural and artificial cavities, and a solution comprising at least one lipophilic flavouring and at least one lipophilic component, the lipophilic solution being applied to the cavities of the fibrous material. The flavour release material is contemplated for bringing about a controlled release of a flavouring in a food matrix, which implies a release of the individual flavour chemicals (flavourings) when release is desired, i.e. not to a greater extent by chemical interactions with other ingredients in the solution or by evaporation when heated during food processes. Controlled release is said to be achieved when the flavour release material according to WO07123466 A1 is exposed to mechanical action, for instance chewing, since the flavourings then migrate from the fat phase into the aqueous phase (saliva) in the oral cavity as the vegetable fibrous material is disintegrated. As is mentioned in WO07123466 A1, the flavour release material is said to have the advantage of being able to accomplish controlled release of flavourings. Since the flavourings are protected in the cavities of the fibers in the material by means of lipophilic components, the migration of the flavourings into the solution is restricted.

An object of the present invention is to provide a new type of system which can be used in aqueous matrices in end products, such as foodstuff, food supplements, chemico-technical products or pharmaceutical preparations, so that lipophilic components may be distributed evenly in hydrophilic matrices. The system aims at functioning as a facilitator for mixing in and protect certain components while using the material at a working up application for the production of an end product, but also aims at releasing such components in a controlled way, such as by delayed or sustained release, at the use of the end product. Another purpose of the present invention is to provide different types of applications, such as use of the emulsifier system and various types of products comprising the emulsifier system.

### Summary of the Invention

The first object given above is achieved by an emulsifier system comprising a fiber system including cellulose, hemicellulose and starch, and wherein the emulsifier system also comprises at least one lipophilic component, wherein the fiber part of the fiber system is insoluble in water and oil, wherein the emulsifier system comprises a water phase and a lipophilic phase where the lipophilic phase is emulsified in the water phase, wherein the fiber acts as a support phase which renders it possible to evenly disperse lipophilic components in the water phase, and wherein the emulsifier system is in the form of a paste having a viscosity to hold water insoluble particles suspended in the paste.

As is known, an emulsion is a mixture of two liquids that normally don't mix easily, e.g. water and oil or fat. An emulsion is produced by breaking a substance into such small droplets that they float freely in the surrounding liquid, i.e. in the other substance. This can be done in various ways, which results in that there are various types of emulsions, such as emulsions of the type "water in oil" and the type "oil in water". An emulsifier may thus be defined as a substance that is used to achieve a uniform mixture of a product, such as an emulsion, which consists of two or more components, where several components don't easily mix with each other, such as an oil phase in a water phase according to the above. Further, as an explanation, an emulsifier consists of water soluble hydrophilic components and water-insoluble, oil/fat-soluble lipophilic (hydrophobic) parts. When an emulsifier is added to a mixture of water and oil the emulsifier will be placed at the interface between these so that the hydrophilic part of the emulsifier is anchored in the water phase and the lipophilic part is anchored in the oil/fat-phase.

As is notable from the expression "where the lipophilic phase is emulsified in the water phase", the system according to the present invention is directed to an "oil-in-water" system.

Furthermore, the expression "and wherein the emulsifier system is in the form of a paste having a viscosity to hold water insoluble particles suspended in the paste" is essential according to the present invention. This feature is directed to both the actual form of the system, i.e. not being a liquid or a total solid form, and the properties of the paste as such. As the paste has a viscosity to hold water insoluble particles suspended in the paste no such insoluble particles are found floating on the surface as floccules or found sedimented on the bottom of a container holding the paste. Therefore, the viscosity of the paste must be sufficient to stabilise the system and avoid separation or transport of insoluble particles to the surface.

In WO2012082065 A1 there is disclosed a particle stabilized emulsion or foam comprising at least two phases and solid particles. The solid particles are starch granules and said starch granules or a portion thereof is situated at the interface between the two phases providing the particle stabilized emulsion or foam. There are no fibers or fiber system disclosed or hinted in WO2012082065 A1. Furthermore, cellulose or hemicellulose is not components which are relevant in relation to the solution disclosed in WO2012082065 A1. The present invention, however, is directed to an emulsifier system comprising a fiber system including cellulose, hemicellulose and starch EP 714609 A2 discloses a whipping cream comprising an emulsifier system consisting of lecithin and polyglycerol esters.

WO 2012/099527 A1 discloses an emulsifier in powder or paste form comprising at least cellulose, hemicellulosde or starch.

US 4980193 is directed to stabilizing agents useful as dispersants comprising microcrystalline cellulose and starch.

In XP 90485544 (Food Sci. Biotech. Vol. 10, No. 4, pp. 348-353 of 2001) the stabilization effect of dieatary fiber from corn hull is described.

Moreover, in US 5676994 there is disclosed a method of preparing a composition characterized by a uniform and stable distribution of lipid throughout a continuous starch phase, the method comprising co-cooking an aqueous dispersion of starch and a lipid under conditions which will completely solubilize the starch so as to form an aqueous solution of starch in the non-retrograded state and under conditions of sufficient turbulence to produce an emulsion comprising droplets of said lipid uniformly dispersed throughout the aqueous starch solution; and recovering the resultant emulsion under conditions which stabilize the distribution of the lipid in the starch phase. In US 5676994 there are no fibers or fiber systems disclosed. Furthermore, also in this case cellulose or hemicellulose is not components which are part of the solution disclosed in US 5676994.

Once again, the present invention, however, is directed to an emulsifier system comprising a fiber system including cellulose, hemicellulose and starch,

### Specific Embodiments of the Invention

The emulsifier system according to the present invention may be depicted to be comprised in different variants of end products. Examples are free flowing powders or pure paste forms or emulsions. Examples of the latter are soft ice cream and paints. These two latter products contain particles suspended in a paste or emulsion, and need some kind of forced separation like centrifugation for separation of the two different phases. One of the parameters differing when going from a free flowing powder to a paste is the content of lipophilic phase. According to the present invention, the lipophilic phase constitutes a maximum of 60 wt% of the emulsifier system. This is fact both covers the free flowing powders having a lipophilic content of up to about 30 wt% as well as the pure pastes having a lipophilic content of up to about 60 wt%. According to one specific embodiment of the present invention, the lipophilic phase constitutes from 20 to 60 wt% of the emulsifier system. This range covers most of the interesting applications according to the present invention.

As mentioned above, the emulsifier system according to the present invention comprises at least one lipophilic component. According to one specific embodiment, said at least one lipophilic component is an oil, e.g. a vegetable oil. Other possible examples are mixtures of different oils or one or several monoglycerides, or the like.

Other components may also be comprised in the emulsifier system according to the present invention. According to one specific embodiment, the emulsifier system also comprises at least one protective barrier formation component for increasing the storage stability. According to one specific embodiment, the at least one protective barrier formation component is added to the emulsifier system. This addition may for instance be performed as a coating of the emulsifier system, such as applied by a coating technique. The protective barrier formation component may according to one embodiment be a dextrose polymer, e.g. different forms of dextrin or starch derivatives, saccharose, fat or a plastic polymer, or a derivative thereof, or a combination thereof. The addition of components like these is made for protecting lipophilic parts against oxidation.

According to one specific embodiment of the present invention, the at least one protective barrier formation component, or the combination of several such, constitutes from 70 to 90 wt% of the emulsifier system.

The fibrous material according to the present invention may be derived from various materials of origin. In principle may many possible starting materials of the fibrous material be used, such as fibrous material based on potato, beetroot, pea, tapioca, carrot or parsnip. Mixtures of these are also possible and also other types of fibrous materials can be used. Potato raw material is one important variant as a fiber material according to the present invention.

Furthermore, some proportions, such as the starch percentage, may vary, e.g. based on the intended application, but it is also possible that this level is enriched in the system, such as according to above. The starch will always be present since it is of importance for the emulgating effect in aqueous phase in end products according to the present invention, on one hand by swelling and on the other hand by pasting. Variants of such end products comprising the system according to the present invention are discussed more below. Since starch may be present in both bound form and free form and at different proportions, the pasting and thereby the viscosity may be controlled. This in turn controls the distribution rate in an aqueous medium. By controlling the degree of pasting the temperature for release may be controlled. Totally swelled starch gives maximum viscosity at room temperature while unswelled starch gives a low viscosity at room temperature. The homogenity is further controlled via the starch and the viscosity of the hydrophilic phase in the end product (see below), which partly is controlled by the viscosity of the starch in the fibrous raw material.

The emulsifier system according to the present invention may be used in many types of applications or products (emulsified systems). The products may for instance be foodstuff, food supplements, such as food colouring, chemico-technical products, such as paint and e.g. pharmaceutical preparations. For paints is above all a fat-soluble food colouring of interest, but also regular paint is a possible end product in which the emulsifier system may be contained. According to one specific embodiment, a product comprising an emulsifier system according to the present invention is contemplated, wherein the product is chosen from one of a pharmaceutical preparation, a foodstuff, food supplement or a chemico-technical product and wherein the active component being liposoluble is active with regard to the product, and wherein the emulsifier system promotes controlled release of the active component. In the case of for instance a pharmaceutical preparation this may be accomplished in the stomach by the emulsifier system and saliva.

With active lipophilic component is contemplated, for a food supplement, e.g. different types of vitamins, sweeteners, but also e.g. colouring, such as in a food colouring according to the present invention, for a pharmaceutical preparation an active substance for the contemplated indication, i.e. a pharmaceutical substance, and for a chemico-technical product a substance which is active for the contemplated use, such as a colorant/pigment for a paint substance. Mixtures of a number of so called active components are also possible according to the present invention, i.e. in for example a foodstuff or a food supplement there may be colorants as well as some kind of active pharmaceutical component present.

The present system has a number of advantages in products according to the above. Firstly, the system brings about a protective effect, such as against oxidation, since the oil phase and the fibrous matrix protect against air, for one or more active components defined according to the above. The active component/components follow the lipophilic phase for incorporation or encapsulation in the cavities of the fibrous material. This results in that the release of the active component/components may be controlled, such as delayed, prolonged or hindered. Controlled release may refer to the release of flavourings by mechanical action or decomposition of a foodstuff. For example when a foodstuff comprising the emulsifier system and the fibrous material is chewed and thereby decomposed, incorporated flavourings may be released. This effect is the same type of effect that is described for the decomposition of the flavour release material according to WO07123466 A1.

Delayed or prolonged release may for instance be of interest at the incorporation of active pharmaceutical substances. It is also possible that such active substances may be incorporated in a food product, such as an emulsion. When eating such a foodstuff it is possible to ensure that a portion of the active substance that is big enough is released in the stomach. Such emulsion pharmaceuticals or other solid types of pharmaceutical products that are not foodstuffs, but are intended for oral consumption, are also possible according to the present invention. By using an emulsifier system according to the present invention in such products a similar effect may be achieved as is achieved when gelatine capsules are used today, i.e. a protective effect for an active substance which is to be released in e.g. the stomach. To further strengthen such an effect, and to fully imitate a gelatine capsule intended to comprise pharmaceutical substances, a product according to the present invention may also be coated, for instance with a dextrose polymer. Therefore, according to one embodiment of the present invention, there is disclosed an emulsified system having a coating of a dextrose polymer, for promoting slow release, such as when containing a pharmaceutical preparation as the active component in an emulsified product. The slow release profile may be controlled by the amount of fiber and starch in relation to active component. In addition to giving e.g. "slow release" for a pharmaceutical substance also taste masking can be achieved using an emulsifier system according to the present invention. For instance when certain active components, such as pharmaceutical components, have a rank or unpleasant taste, the emulsifier system according to the present invention may result in that the consumer doesn't feel the taste of these as much, or at all, as the release can take place fully in for instance the stomach. In that way a similar effect can be achieved which otherwise is accomplished in products by coating of the substances the taste of which is to be masked. As an example antibiotics may be mentioned as a suitable substance for masking of the taste by the incorporation in a product comprising an emulsifier system according to the present invention. Furthermore, the release may be delayed in comparison to a regular tablet formulation comprising antibiotics today.

Active substances in a pharmaceutical application according to the present invention comprises dietary supplements, such as vitamins, minerals and other wholesome supplements, such as omega 3-fatty acid or omega 6-fatty acid etcetera, but of course also pharmaceutical substances which today are found in pharmaceuticals in capsules or tablets or the like.

In a colour according to the present invention the at least one active component is a colorant. The colour product may be a paint product as well as a food colouring.

In addition to various application types the products may also have various physical nature. According to one embodiment the product is an emulsion of the type oil-in-water. Such an emulsion may for instance be a paint or a foodstuff, but also a pharmaceutical preparation as mentioned above. The emulsions or emulsified systems obtainable according to the present invention consist of three phases, which phases are water, oil and fibrous material, where the fibrous material is the base in the paste and is considered a support phase for accomplishing the emulsion. As is realised from above, the end products, for instance a pharmaceutical preparation, foodstuff or colorant, according to the present invention also have a hydrophilic phase. Consequently, it is the emulsifier system with its fibrous part (support phase) and lipophilic phase that is added to an aqueous matrix for the production of these end products according to the present invention.

The active components are mixed in the lipophilic phase. The lipophilic phase is protected inside the cavities of the fibrous material. By this protection of active components, the emulsifier system comprising the fibrous material and its lipophilic phase holding the active components may thereafter be added to an aqueous system, dispersing the lipophilic phase in the water phase, resulting in that the lipophilic part with its active components is protected by the fibrous material and the produced emulsion and thereby is not dissolved out into the aqueous phase. The protection also applies against oxidation, which is mentioned above.

As is mentioned above, different types of physical end products are possible to produce using the system according to the present invention. This means that not only end products in the form of emulsions are possible. Different types of end products have in common that water is present at some point during the processing. The water content may, however, vary for different applications, both during the processing and in the end product.

For instance, it is possible that the water content is very low in some foodstuff end products according to the present invention, for instance in certain snacks, but where the water content has been higher during the food process for the production of the snacks. When heating by for instance roasting, boiling or drying is present at the production of a foodstuff the water content is lowered. The system according to the present invention is in these cases especially important during the processing to give the end product the intended taste and/or colour. By introducing the third phase consisting of the fibrous material in an emulsifier system according to the present invention the mixture of water/oil is controlled and stabilised before it is further processed.

Also described according to the present invention is the use of an emulsifier system according to the invention. According to one specific embodiment the use of an emulsifier system according to the present invention is contemplated for the creation of an emulsion of the type oil-in-water. As is mentioned above, this use of lipophilic components that are comprised may be evenly dispersed in an aqueous system, i.e. in a hydrophilic matrix.

As is described above, even if an aqueous phase is comprised during the use, the water content may be reduced during the process, for instance by different types of heat treatments or the like. The present invention therefore also contemplates the use of an emulsifier system according to the present invention for the production of a solid product or a product in the form of a paste. All such products may be seen as emulsified systems.

As is mentioned above, typical end products, both solid and emulsion based, are e.g. pharmaceutical preparations, foodstuffs, food supplements, such as food colorants, or chemico-technical products, such as paint substances. The products have in common that at least one active component of the product is incorporated in the lipophilic part that is protected inside the fibrous material. In that way the release of such active components is controlled even if the emulsifier system is mixed with aqueous phase. The present invention therefore also contemplates use of an emulsifier system according to the present invention for the encapsulation of at least one, with reference to the end product, active component.

There are many types of processes for manufacturing that are used today where the emulsifier system according to the present invention is applicable. In for instance foodstuff processes where starch is present, pasting, hydrolysation and dextrination are typical examples of such processes. The emulsifier system according to the present invention is suitable for all these cases. As is mentioned above, the starch content may vary in the system and this also controls the degree of dispersibility. This in turn also controls although pasting is suitable to perform for obtaining so called cold water-soluble products. The emulsifier system according to the present invention may be used for cold water-soluble products as well as hot water-soluble products.

The products may also be hydrolyzed, for instance by enzymatic hydrolysis or common acid hydrolysis. Furthermore, dextrination, i.e. addition of some kind of dextrose polymer through a coating procedure, is possible with products comprising the emulsifier system according to the present invention. As is described above, such a process may be of interest when producing a foodstuff product comprising for instance an active pharmaceutical component or other active component. In that way the delayed release may be strengthened so that in principle all the release of active component takes place in the stomach. In other words may such a product be claimed to imitate a gelatine capsule.

The various process steps described above may be used in series and in various order in for instance a complete foodstuff production. Furthermore is it important to understand that these process steps only are examples, and that also totally different process steps, such as various steps of heat treatment or extrusion, may be included or be used alone with the system according to the present invention.

### Conclusions

The emulsifier system according to the present invention has many advantages. The system is firstly based on totally natural bases of composition, such as vegetable fibrous material and lipophilic phase, e.g. rapeseed oil or other vegetable oils. Furthermore, with the emulsifier system according to the present invention it is possible to affect the release of various types of components that are incorporated in the emulsifier system in some type of end product. Since the fibrous material incorporates the lipophilic phase, such components may be protected also when aqueous phase is added to a product. As is discussed above, controlled release, such as delayed or prolonged release, may be of interest for pharmaceutical components in pharmaceutical products or foodstuff products with such an effect, and for e.g. colouring agents/pigments in food colouring or other types of paint. Also masking of taste, which also can be said to be a delayed release of certain flavourings, may be achieved in products comprising the emulsifier system according to the present invention. Such an effect may also be increased by coating of these end products with some kind of dextrose polymer. Such a component or other components like saccharose, fat or plastic polymers may be added to the system for increasing the storage stability, in fact in a level of up to from 70 to 90 wt% of the total of the emulsifier system.

## Claims

1. Emulsifier system comprising a fiber system including cellulose, hemicellulose and starch, and wherein the emulsifier system also comprises at least one lipophilic component, wherein the fiber part of the fiber system is insoluble in water and oil, wherein the emulsifier system comprises a water phase and a lipophilic phase where the lipophilic phase is emulsified in the water phase, wherein the fiber acts as a support phase which renders it possible to evenly disperse lipophilic components in the water phase, and wherein the emulsifier system is in the form of a paste having a viscosity to hold water insoluble particles suspended in the paste, wherein the lipophilic phase constitutes a maximum of 60 wt% of the emulsifier system.

2. Emulsifier system according to claim 1, wherein said at least one lipophilic component is an oil.

3. Emulsifier system according to any of claims 1 or 2, wherein the lipophilic phase constitutes from 20 to 60 wt% of the emulsifier system

4. Emulsifier system according to any of claims 1-3, wherein the emulsifier system also comprises at least one protective barrier formation component for increasing the storage stability.

5. Emulsifier system according to claim 4, wherein the at least one protective barrier formation component is a dextrose polymer, saccharose, fat or a plastic polymer, or a derivative thereof, or a combination thereof.

6. Emulsifier system according to any of claims 4-5, wherein the at least one protective barrier formation component, or the combination of several such, constitutes from 70 to 90 wt% of the emulsifier system.

7. Emulsifier system according to any of claims 1-6, wherein the vegetable fiber is based on potato, beetroot, pea, tapioca, carrot or parsnip, or a combination thereof.

8. Emulsifier system according to claim 7, wherein the vegetable fiber is based on potato raw material.

9. Product comprising an emulsifier system according to any of claims 1-8, wherein the product is a pharmaceutical preparation, a foodstuff, a food supplement or a chemico-technical product and wherein the active component being liposoluble is active with regard to the product, and wherein the emulsifier system promotes controlled release of the active component.

10. Pharmaceutical preparation comprising an emulsifier system according to any of claims 1-8, wherein the pharmaceutical preparation also comprises at least one active pharmaceutical component.

11. Foodstuff comprising an emulsifier system according to any of claims 1-8.

12. Foodstuff supplement comprising an emulsifier system according to any of claims 1-8, wherein the foodstuff supplement also comprises at least one vitamin and/or a sweetener and/or a colorant and/or an active pharmaceutical component.

13. Chemico-technical product comprising an emulsifier system according to any of claims 1-8.

14. Use of an emulsifier system according to any of claims 1-8 for the production of an emulsion, a solid product or a product in the form of a paste.

## Patentansprüche

1. Emulgatorsystem umfassend ein Fasersystem, das Cellulose, Hemicellulose und Stärke enthält, und wobei das Emulgatorsystem auch mindestens eine lipophile Komponente umfasst, wobei der Faserteil des Fasersystems in Wasser und Öl unlöslich ist, wobei das Emulgatorsystem eine Wasserphase und eine lipophile Phase umfasst, wobei die lipophile Phase in der Wasserphase emulgiert ist, wobei die Faser als Trägerphase dient, die es ermöglicht, dass die lipophilen Komponenten gleichmäßig in der Wasserphase dispergiert werden, und wobei das Emulgatorsystem in Form einer Paste vorliegt, die eine derartige Viskosität aufweist, dass die wasserunlöslichen Teilchen in der Paste suspendiert gehalten werden, wobei die lipophile Phase maximal 60 Gew.-% des Emulgatorsystems ausmacht.

2. Emulgatorsystem nach Anspruch 1, wobei die mindestens eine lipophile Komponente ein Öl ist.

3. Emulgatorsystem nach einem der Ansprüche 1 oder 2, wobei die lipophile Phase 20 bis 60 Gew.-% des Emulgatorsystems ausmacht.

4. Emulgatorsystem nach einem der Ansprüche 1 bis 3, wobei das Emulgatorsystem auch mindestens eine Schutzbarrieren-Bildungskomponente zur Erhöhung der Lagerstabilität umfasst.

5. Emulgatorsystem nach Anspruch 4, wobei die mindestens eine Schutzbarrieren-Bildungskomponente ein Dextrosepolymer, Saccharose, Fett oder ein Kunststoffpolymer oder ein Derivat davon oder eine Kombination davon ist.

6. Emulgatorsystem nach einem der Ansprüche 4 bis 5, wobei die mindestens eine Schutzbarrieren-Bildungskomponente oder die Kombination von mehreren davon 70 bis 90 Gew.-% des Emulgatorsystems ausmacht.

7. Emulgatorsystem nach einem der Ansprüche 1 bis 6, wobei die Pflanzenfaser auf Kartoffel, Rote Bete, Erbse, Tapioka, Karotte oder Pastinake oder einer Kombination davon basiert.

8. Emulgatorsystem nach Anspruch 7, wobei die Pflanzenfaser auf Kartoffelrohmaterial basiert.

9. Produkt, umfassend ein Emulgatorsystem nach einem der Ansprüche 1 bis 8, wobei das Produkt eine pharmazeutische Zubereitung, ein Nahrungsmittel, ein Nahrungsergänzungsmittel oder ein chemisch-technisches Produkt ist und wobei der fettlösliche Wirkstoff in Bezug auf das Produkt aktiv ist, und wobei das Emulgatorsystem die kontrollierte Freisetzung des Wirkstoffs fördert.

10. Pharmazeutische Zubereitung, umfassend ein Emulgatorsystem nach einem der Ansprüche 1 bis 8, wobei die pharmazeutische Zubereitung auch mindestens einen pharmazeutischen Wirkstoff umfasst.

11. Nahrungsmittel, umfassend ein Emulgatorsystem nach einem der Ansprüche 1 bis 8.

12. Nahrungsergänzungsmittel, umfassend ein Emulgatorsystem nach einem der Ansprüche 1 bis 8, wobei das Nahrungsergänzungsmittel auch mindestens ein Vitamin und/oder einen Süßstoff und/oder einen Farbstoff und/oder einen pharmazeutischen Wirkstoff umfasst.

13. Chemisch-technisches Produkt, umfassend ein Emulgatorsystem nach einem der Ansprüche 1 bis 8.

14. Verwendung eines Emulgatorsystems nach einem der Ansprüche 1 bis 8 zur Herstellung einer Emulsion, eines festen Produkts oder eines Produkts in Form einer Paste.

## Revendications

1. Système émulsifiant comprenant un système de fibres comprenant de la cellulose, de l'hémicellulose et de l'amidon, le système émulsifiant comprenant également au moins un composant lipophile, la partie fibres du système de fibres étant insoluble dans l'eau et l'huile, le système émulsifiant comprenant une phase aqueuse et une phase lipophile, la phase lipophile étant émulsifiée dans la phase aqueuse, la fibre servant de phase de support qui rend possible de disperser de façon uniforme les composants lipophiles dans la phase aqueuse et le système émulsifiant étant sous la forme d'une pâte ayant une viscosité permettant de maintenir des particules insolubles dans l'eau en suspension dans la pâte, la phase lipophile constituant un maximum de 60 % en poids du système émulsifiant.

2. Système émulsifiant selon la revendication 1, ledit au moins un composant lipophile étant une huile.

3. Système émulsifiant selon l'une quelconque des revendications 1 ou 2, la phase lipophile constituant de 20 à 60 % en poids du système émulsifiant.

4. Système émulsifiant selon l'une quelconque des revendications 1-3, le système émulsifiant comprenant également au moins un composant de formation de barrière protectrice pour l'augmentation de la stabilité au stockage.

5. Système émulsifiant selon la revendication 4, l'au moins un composant de formation de barrière protectrice étant un polymère de dextrose, le saccharose, de la matière grasse ou un polymère plastique, ou un dérivé de ceux-ci ou une association de ceux-ci.

6. Système émulsifiant selon l'une quelconque des revendications 4-5, l'au moins un composant de formation de barrière protectrice, ou l'association de plusieurs de tels composants, constituant de 70 à 90 % en poids du système émulsifiant.

7. Système émulsifiant selon l'une quelconque des revendications 1-6, la fibre végétale étant à base de pomme de terre, de betterave, de pois, de tapioca, de carotte ou de panais ou d'une association de ceux-ci.

8. Système émulsifiant selon la revendication 7, la fibre végétale étant à base de matière première issue de pomme de terre.

9. Produit comprenant un système émulsifiant selon l'une quelconque des revendications 1-8, le produit étant une préparation pharmaceutique, une denrée alimentaire, un complément alimentaire ou un produit chimico-technique et le composant actif qui est liposoluble étant actif en ce qui concerne le produit et le système émulsifiant favorisant la libération contrôlée du composant actif.

10. Préparation pharmaceutique comprenant un système émulsifiant selon l'une quelconque des revendications 1-8, la préparation pharmaceutique comprenant également au moins un principe actif pharmaceutique.

11. Denrée alimentaire comprenant un système émulsifiant selon l'une quelconque des revendications 1-8.

12. Complément alimentaire comprenant un système émulsifiant selon l'une quelconque des revendications 1-8, le complément alimentaire comprenant également au moins une vitamine et/ou un édulcorant et/ou un colorant et/ou un principe actif pharmaceutique.

13. Produit chimico-technique comprenant un système émulsifiant selon l'une quelconque des revendications 1-8.

14. Utilisation d'un système émulsifiant selon l'une quelconque des revendications 1-8 pour la production d'une émulsion, d'un produit solide ou d'un produit sous la forme d'une pâte.
